# EUROPEAN PATENT SPECIFICATION

(11) **EP 1 974 671 B1**
(45) Date of publication and mention of the grant of the patent: **23.02.2011**
(21) Application number: 08005569.2
(22) Date of filing: 26.03.2008
(51) Int. Cl.: A61B 8/00, G06F 3/01, G06F 19/00, A61B 17/00

(54) **Ultrasound system**
Ultraschallsystem
Système à ultrasons

(30) Priority: 27.03.2007 KR 20070029494
(43) Date of publication of application: 01.10.2008
(73) Proprietor: MEDISON CO., LTD., Kangwon-do 250-870 (KR)
(72) Inventor: Shin, Dong Kuk, Seoul 135-280 (KR)
(74) Representative: Lorenz, Markus

(56) References cited:
- WO-A-2006/087689
- US-A- 5 544 654
- US-A1- 2002 128 846
- US-A1- 2005 025 706
- TRIESCH J ET AL: "Robust classification of hand postures against complex backgrounds" PROCEEDINGS OF THE 1996 SECOND INTERNATIONAL CONFERENCE ON AUTOMATIC FACE AND GESTURE RECOGNITION, KILLINGTON, VT, USA, 14-16 OCT. 1996, LOS ALAMITOS, CA, USA, IEEE COMPUT. SOC, US, 14 October 1996 (1996-10-14), pages 170-175, XP010200416 ISBN: 978-0-8186-7713-7
- KOLSCH M ET AL: "Robust hand detection" AUTOMATIC FACE AND GESTURE RECOGNITION, 2004. PROCEEDINGS. SIXTH IEEE INTERNATIONAL CONFERENCE ON, IEEE, PISCATAWAY, NJ, USA, 17 May 2004 (2004-05-17), pages 614-619, XP010949501 ISBN: 978-0-7695-2122-0

## Description

The present application claims priority from Korean Patent Application No. 10-2007-0029494 filed on March 27, 2007.

### BACKGROUND OF THE INVENTION

### [Technical Field]

The present invention generally relates to ultrasound systems, and more particularly to an ultrasound system configured to recognize the action of a user.

### [Background Art]

The ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

Generally, the ultrasound system includes an input unit for receiving various inputs from a user. The input unit may include a control panel for allowing the user to select a menu. The control panel may contain a touch screen displaying the menu for image optimization and providing a function of selecting the menu, a track ball for inputting a moving instruction to move a cursor on a screen of the display unit, a keyboard for inputting texts and providing shortcut keys in various measurement modes, etc. The control panel is typically mounted on the ultrasound system and may be movable in the up, down, right and left directions.

However, the control panel may be movable within a limited range in the conventional ultrasound system. Thus, the user may manipulate the control panel in an uncomfortable posture while the user grasps the probe to examine a patient. Also, when the ultrasound system is far from the user, it is difficult to manipulate the control panel.

US 5,544,654 describes a method and an apparatus for voice activation of an ultrasound system having a plurality of voice commands. A structured vocabulary where subsets of a total large vocabulary are used for speech recognition is used to control an ultrasound machine. Furthermore, the total vocabulary is divided into smaller sub-vocabularies for maximizing the control and the recognition performance.

From XP010200416 "Robust classification of hand postures against complex backgrounds" from Jochen Triesch and Christoph von der Malsburg it is known to increase the recognition of the gestures by employing elastic graph matching. The procedure described therein is completely different from US 5,544,654.

### BRIEF DESCRIPTION OF THE DRAWINGS

FIG. 1 is a block diagram showing an ultrasound system constructed in accordance with one embodiment of the present invention.

FIG. 2 is diagram showing an example of associating training sets corresponding to actions with instructions for operating the ultrasound system constructed in accordance with one embodiment of the present invention.

### DETAILED DESCRIPTION OF THE INVENTION

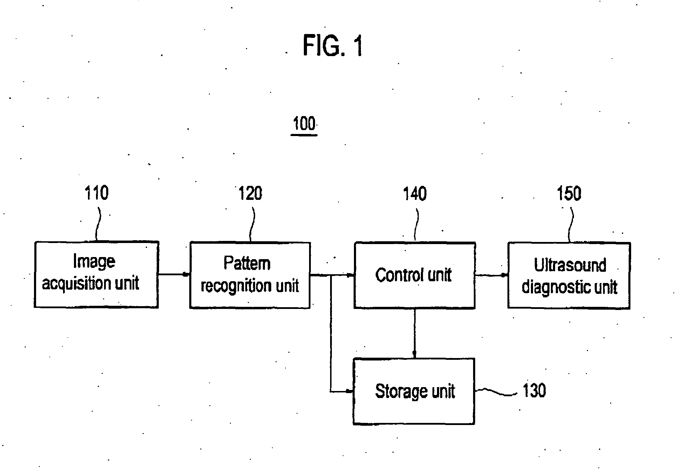

FIG. 1 is a block diagram showing an ultrasound system constructed in accordance with one embodiment of the present invention. Referring to FIG. 1, the ultrasound system 100 includes an image acquisition unit 110, an image processing unit 120, a storage unit 130, a control unit 140 and an ultrasound diagnostic unit 150.

The image acquisition unit 110 may be operable to capture various actions of a user to acquire action images. A plurality of action images for the same action may be acquired in different angles. The action images may be a digital image. The image acquisition unit 110 may be any type of imaging devices capable of acquiring digital images such as a digital camera.

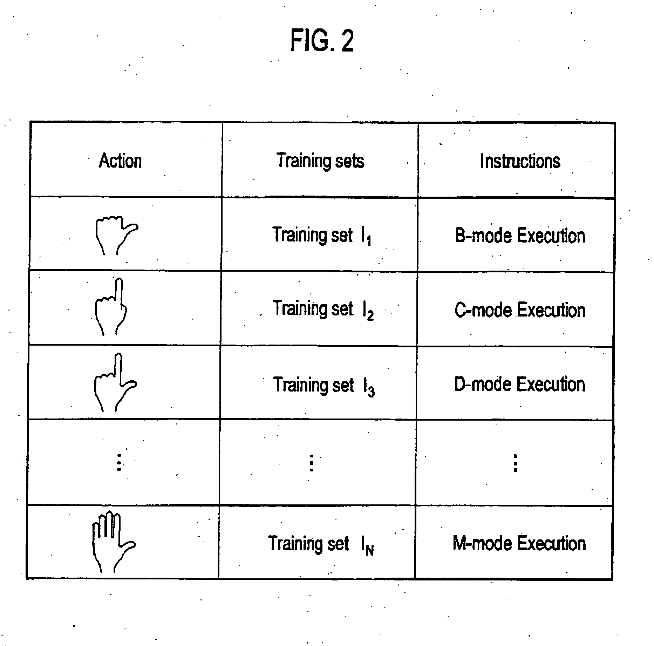

The pattern recognition unit 120 may be operable to recognize action patterns from the acquired images. The pattern recognition unit 120 may perform segmentation upon the action images to extract a specific portion (e.g., a user's hand). The pattern recognition unit 120 may simplify the extracted portion to thereby obtain pattern samples of the user actions. The simplification may be achieved by indicating boundaries in the extracted portion with chain codes or performing skeleton extraction upon the extracted portion. The pattern recognition unit 120 may generate training sets with the pattern samples for the respective actions, which may indicate gestures of the user's hand in accordance with one embodiment of the present invention.. The pattern recognition unit 120 may form a table in which the training sets are associated with respective instructions for operating the ultrasound system 100, as shown in FIG. 2. The corresponding relations of the training sets and the instructions may be arbitrarily set by the user The mapping table may be stored in the storage unit 130.

While the ultrasound system. 100 operates, the image acquiring unit 120 may repeatedly acquire an action image for the user. The acquired action image is provided to the pattern recognition unit 120. The pattern recognition unit 120 may extract a portion corresponding to the user's hand through segmentation and recognize an action pattern from the extracted portion.

The control unit 140 may be operable to retrieve the training set stored in the storage unit 130 to find a pattern sample matched with the recognized action pattern in the training sets. The control unit 140 may control the ultrasound diagnostic unit 150 based on an instruction associated with the training set in which the found pattern sample is included. For example, if it is determined that the found pattern sample is included in the first training set I₁, then the control unit 140 may detect the instruction of B-mode execution and control the ultrasound diagnostic unit 150 to operate in the B-mode.

Although the action images of the user's hand are used to form pattern samples in accordance with one embodiment of the present invention, various action images for different portions of the user (e.g., a head, a leg, etc.) may be used in accordance with another embodiment of the present invention.

The ultrasound diagnostic unit 150 may be operable to transmit ultrasound signals to a target object and receive ultrasound echo signals to thereby form an ultrasound image of the target object under the control of the control unit 140. The ultrasound diagnostic unit 150 may include a probe, a beam former, a signal processing unit, an image processing unit, a display unit and an input unit The probe may transmit and receive ultrasound signals The transducer elements may generate the ultrasound signals in response to transmission pulse signals and electrical reception signals in response to ultrasound echo signals reflected from discontinuous planes of acoustic impedance in the target object. The transducer elements may individually transmit the ultrasound signals or several transducer elements may transmit the ultrasound signals at the same time.

The beam former may form a transmission pattern of the transmission pulse signals such that the ultrasound signals generated from the transducer elements are focused on a focal point. Also, the beam former may focus the electrical reception signals based on distances between the transducer elements and the focal point.

The signal processing unit may perform analog-to-digital conversion, amplification and various signal process upon the focused reception signals. The image processing unit may form an ultrasound image based on the processed signals. The display unit may display the ultrasound image.

The imaging acquisition unit 110, the image reorganization unit 120, the storage unit 130 and the control unit 140 may be configured into a computing device such as a personal computer. The computing device may be interconnected with the ultrasound diagnostic unit 150 through various communication networks. The computing device may remotely control the ultrasound diagnostic unit 150 by using the Internet.

As described above, since the ultrasound diagnostic unit may operate in response to the actions of the user, the user may comfortably operate the ultrasound diagnostic unit.

In accordance with one embodiment of the present invention, there is provided an ultrasound system, which includes the following an ultrasound diagnostic unit for forming an ultrasound image representative of a target object; a storage unit for storing training sets in association with respective instructions for operating the ultrasound diagnostic unit, each of said training sets including pattern samples associated with a particular action of a user; an image acquisition unit for capturing an action of a user to form an action image; a pattern recognition unit for recognizing an action pattern from the action image; and a control unit for retrieving the training sets stored in the storage unit to find a pattern sample having a match with the recognized action pattern, the control unit being configured to control an operation of the ultrasound diagnostic unit based on an instruction associated with the training set including the found pattern sample.

## Claims

1. An ultrasound system (100) for displaying ultrasound images, comprising:
an ultrasound diagnostic unit (150) for forming an ultrasound image representative of a target object;
**characterized by** further comprising:
a storage unit (130) for storing training sets in association with respective instructions for operating the ultrasound diagnostic unit, each of said training sets including pattern samples associated with a particular action of a user;
an image acquisition unit (110) for capturing an action of a user to form an action image;
a pattern recognition unit (120) for recognizing an action pattern from the action image; and
a control unit (140) for retrieving the training sets stored in the storage unit (130) to find a pattern sample having a match with the recognized action pattern, the control unit (140) being configured to control an operation of the ultrasound diagnostic unit (150) based on an instruction associated with the training set including the found pattern sample.

2. The ultrasound system (100) of Claim 1, wherein the pattern samples are obtained by capturing various actions of the user in different angles by the image acquisition unit

3. The ultrasound system (100) of Claim 2, wherein the action of the user is one of actions of a hand, a head and a leg.

4. The ultrasound system (100) of Claim 1, wherein the control unit (140) controls the ultrasound diagnostic unit through the Internet.

## Patentansprüche

1. Ultraschallsystem (100) zum Anzeigen von Ultraschallbildern, welches Folgendes aufweist:
eine Ultraschalldiagnoseeinheit (150) zum Bilden eines für ein Zielobjekt repräsentativen Ultraschallbilds;
**gekennzeichnet durch:**
eine Speichereinheit (130) zum Speichern von Trainingssätzen in Verbindung mit entsprechenden Anweisungen zum Betreiben der Ultraschalldiagnoseeinheit, wobei jeder der Trainingssätze Modellmuster aufweist, welche mit einer bestimmten Tätigkeit eines Nutzers in Verbindung stehen;
eine Bildbeschaffungseinheit (110) zum Erfassen einer Tätigkeit eines Benutzers, um ein Tätigkeitsbild zu bilden;
eine Mustererkennungseinheit (120) zum Erkennen eines Tätigkeitsmusters aus dem Tätigkeitsbild; und
eine Steuereinheit (140) zum Abfragen der in der Speichereinheit (130) gespeicherten Trainingssätze, um ein Modellmuster zu finden, welches mit dem erkannten Tätigkeitsmuster zusammenpasst, wobei die Steuereinheit (140) so ausgebildet ist, dass sie den Betrieb der Ultraschalldiagnoseeinheit (150) basierend auf einer mit dem Trainingssatz, welcher das aufgefundene Modellmuster einschließt, in Verbindung stehenden Anweisung steuert.

2. Ultraschallsystem (100) nach Anspruch 1, wobei die Modellmuster durch Erfassen unterschiedlicher Tätigkeiten des Benutzers in unterschiedlichen Winkeln durch die Bildbeschaffungseinheit erhalten werden.

3. Ultraschallsystem (100) nach Anspruch 2, wobei die Tätigkeit des Benutzers eine Bewegung einer Hand, eines Kopfs oder eines Beins ist.

4. Ultraschallsystem (100) nach Anspruch 1, wobei die Steuereinheit (140) die Ultraschalldiagnoseeinheit durch das Internet steuert.

## Revendications

1. Système à ultrasons (100) pour l'affichage d'images ultrasonores, comprenant :
une unité de diagnostic ultrasonore (150) pour former une image ultrasonore représentative d'un objet cible;
**caractérisé en ce qu'**il comprend en outre :
une unité de stockage (130) pour stocker des ensembles d'apprentissage en association avec des instructions respectives pour faire fonctionner l'unité de diagnostic ultrasonore, chacun desdits ensembles d'apprentissage comprenant des échantillons de forme associés à une action particulière d'un utilisateur;
une unité d'acquisition d'image (110) pour capturer une action d'un utilisateur pour former une image d'action;
une unité de reconnaissance de forme (120) pour reconnaître une forme d'action à partir de l'image d'action; et
une unité de commande (140) pour extraire les ensembles d'apprentissage stockés dans l'unité de stockage (130) pour trouver un échantillon de forme ayant une correspondance avec la forme d'action reconnue, l'unité de commande (140) étant configurée pour commander une opération de l'unité de diagnostic ultrasonore (150) sur la base d'une instruction associée à l'ensemble d'apprentissage incluant l'échantillon de forme trouvé.

2. Système à ultrasons (100) de la revendication 1, dans lequel les échantillons de forme sont obtenus en capturant plusieurs actions de l'utilisateur sous différents angles par l'unité d'acquisition d'image.

3. Système à ultrasons (100) de la revendication 2, dans lequel l'action de l'utilisateur est l'une des actions d'une main, d'une tête et d'une jambe.

4. Système à ultrasons (100) de la revendication 1, dans lequel l'unité de commande (140) commande l'unité de diagnostic ultrasonore à travers l'Internet.
